# EUROPÄISCHE PATENTANMELDUNG

(11) **EP 3 162 364 A1**
(43) Veröffentlichungstag der Anmeldung: **03.05.2017**
(21) Anmeldenummer: 15191941.2
(22) Anmeldetag: 28.10.2015
(51) Int. Cl.: A61K 9/70, A61K 31/465

(54) **VERFAHREN ZUR KENNZEICHNUNG FILMFÖRMIGER DARREICHUNGSFORMEN**

(71) Anmelder: LTS LOHMANN Therapie-Systeme AG, 56626 Andernach (DE)
(72) Erfinder: Botzem, Petra, 56626 Andernach (DE); Grunenberg, Torsten, 56645 Nickenich (DE); Hille, Thomas, 56567 Neuwied (DE); Steinborn, Peter, 56567 Neuwied (DE)

(57) **Zusammenfassung**

Offenbart wird ein Verfahren zum Kennzeichnen filmförmiger Darreichungsformen, umfassend zumindest das Platzieren einer filmförmigen Darreichungsform zwischen zwei Lagen siegelfähigen Packstoffs, das abschnittweise Heißsiegeln der zwei Lagen des siegelfähigen Packstoffs zu einem die filmförmige Darreichungsform enthaltenden Siegelrandbeutel, und das Blindprägen der in dem Siegelrandbeutel enthaltenen filmförmigen Darreichungsform durch zumindest eine der beiden Packstofflagen des Siegelrandbeutels.

## Beschreibung

Die Erfindung betrifft filmförmige Darreichungsformen. Die Erfindung betrifft insbesondere ein Verfahren zur Kennzeichnung von filmförmigen Darreichungsformen, sowie mit diesem Verfahren gekennzeichnete filmförmige Darreichungsformen.

Die Hersteller pharmazeutischer Produkte sind immer häufiger verpflichtet, nicht nur die Primärverpackung eines Arzneimittels mit einem das Produkt identifizierenden Kennzeichen zu versehen, sondern auch die Darreichungsform selbst. Eine Kennzeichnung von festen Darreichungsformen wie Kapseln, Tabletten oder Wafern erfolgt üblicherweise durch deren Bedrucken mit dem Kennzeichen.

Das Bedrucken von oral oder peroral zu verabreichenden, festen Darreichungsformen ist jedoch umstritten und/oder problematisch. So wird beispielsweise diskutiert, ob mit dem Aufbringen der Druckfarbe auf die Darreichungsform ein erhöhtes Allergierisiko für den Patienten durch das Verabreichen der bedruckten Darreichungsform verbunden ist. Bei filmförmigen Darreichungsformen, die einen hohen Anteil öliger Inhaltsstoffe wie Weichmacher oder ätherische Öle enthalten, ist zudem die Verwendung von wasserhaltigen Druckfarben für das Bedrucken nicht möglich, weil diese Druckfarben nicht auf der filmförmigen Darreichungsform halten.

Als Alternative zum Bedrucken von filmförmigen Darreichungsformen wird mit dem europäischen Patent EP 0 949 908 B1 vorgeschlagen, filmförmige Darreichungsformen mit einem Wasserzeichen zu versehen, so dass die filmförmigen Darreichungsformen bei Durchsicht erkennbare Flächenbereiche mit unterschiedlicher Dicke aufweisen. Um filmförmige Darreichungsformen mit einem Wasserzeichen herzustellen sind jedoch besondere Träger nötig, beispielweise strukturierte Unterlagen oder Träger mit unterschiedlich temperierbaren Bereichen.

Eine alternative Möglichkeit zur Kennzeichnung von filmförmigen Darreichungsformen besteht darin, diese mit einer Prägung zu versehen. Allerdings hat sich herausgestellt, dass ein Prägen von filmförmigen Darreichungsformen bevor diese einzeln verpackt werden, nicht zu einer dauerhaften Kennzeichnung der Darreichungsform führt. Die Prägung der filmförmigen Darreichungsform verschwindet durch das für deren Einzelverpacken erforderliche Einsiegeln, weil die für das Einsiegeln einzelner filmförmiger Darreichungsformen in Siegelrandbeutel erforderliche Temperatur von etwa 150 °C bis etwa 200 °C die filmförmige Darreichungsform selbst erweichen lässt, so dass eine zuvor vorgenommene Prägung verschwindet.

Der vorliegenden Erfindung lag daher die Aufgabe zugrunde, einen Weg zu finden, filmförmige Darreichungsformen mit einer Kennzeichnung zu versehen, die auch nach dem Einsiegeln in einen Siegelrandbeutel vorhanden ist und dauerhaft erhalten bleibt.

Diese Aufgabe wird durch ein Verfahren gelöst, bei dem eine bereits eingesiegelte filmförmige Darreichungsform einer Blindprägung unterworfen wird, wobei die Blindprägung der filmförmigen Darreichungsform durch Anwendung von hohem Druck und bei Vermeidung von Wärme, sowie durch den Packstoff hindurch erfolgt.

Überraschenderweise wurde gefunden, dass das Prägen der filmförmigen Darreichungsform durch den Packstoff hindurch zu einer dauerhaften Prägung und somit Kennzeichnung der Darreichungsform führt. Es wird angenommen, dass die mit der Prägung einhergehende Verformung des Packstoffs die Prägung der filmförmigen Darreichungsform so stark stabilisiert, dass diese dauerhaft erhalten bleibt.

Im Sinne der vorliegenden Offenbarung wird unter einer filmförmigen Darreichungsform eine dünne, film-, folien- oder oblatenförmige, mindestens einen Wirkstoff enthaltende Darreichungsformen verstanden, die üblicherweise eine Fläche von etwa 2 cm² bis etwa 8 cm² und eine Dicke im Bereich von etwa 20 µm bis etwa 500 µm aufweist. Der Begriff "filmförmige Darreichungsform" umfasst sowohl einlagige Darreichungsformen wie auch Laminate, bei denen die Darreichungsform zwei, drei oder mehrere Lagen umfasst. Die filmförmige Darreichungsform im Sinne der vorliegenden Offenbarung kann mukoadhäsiv sein. Ferner umfasst "filmförmige Darreichungsform" im Sinne der vorliegenden Offenbarung sowohl den Wirkstoff schnell freisetzende Darreichungsformen, wie auch nach Verabreichung langsam schmelzende oder sich auflösende Darreichungsformen, aber auch den Wirkstoff langanhaltend und annähernd gleichmäßig freisetzende Darreichungsformen. Die filmförmigen Darreichungsformen weisen zumindest eine wirkstoffhaltige Schicht auf, bei der mindestens ein pharmazeutischer Wirkstoff in einem Polymer oder Polymergemisch enthalten ist. Bei mehrschichtigen filmförmigen Darreichungsformen kann die Darreichungsform neben der mindestens einen wirkstoffhaltigen Schicht zumindest eine mukoadhäsive Schicht und/oder eine Rücksicht aufweisen, wobei die Rücksicht für den Wirkstoff impermeabel sein kann.

Bei dem Verfahren erfolgt die Prägung der filmförmigen Darreichungsform am verpackten Produkt, also an der verpackten Darreichungsform. Die Prägung erfolgt durch den Packstoff.

Das Verfahren umfasst die zumindest die folgenden Schritte:
- Platzieren einer filmförmigen Darreichungsform zwischen zwei Lagen siegelfähigen Packstoffs;
- abschnittweises Heißsiegeln der zwei Lagen des siegelfähigen Packstoffs zu einem die filmförmige Darreichungsform enthaltenden Siegelrandbeutel;
- Blindprägen der in dem Siegelrandbeutel enthaltenen filmförmigen Darreichungsform durch zumindest eine der beiden Packstofflagen des Siegelrandbeutels.

Die filmförmige Darreichungsform wird in dem Fachmann auf dem Gebiet der Herstellung filmförmiger Darreichungsformen bekannten Verfahren hergestellt. Diese Verfahren umfassen
- das Mischen der in der Lage oder der in einzelnen Lagen der Darreichungsform enthaltenen Komponenten, wie beispielsweise Polymer oder Polymergemisch, Wirkstoff und optional mindestens einen pharmazeutisch annehmbaren Hilfsstoff, in einem Lösemittel;
- das Ausstreichen beziehungsweise Rakeln der lösemittelhaltigen Masse auf einer Unterlage;
- das Trocknen der ausgestrichenen Masse, um das Lösemittel zu entfernen und einen Polymerfilm zu erhalten;
- das Zerteilen des Polymerfilms in Streifen; und
- das Vereinzeln individueller Darreichungsformen aus den Streifen durch Schneiden oder Stanzen.

Bei dem Verfahren wird eine filmförmige Darreichungsform zwischen zwei Lagen siegelfähigen Packstoffs platziert. Gemäß einer Ausführungsform werden die zwei Lagen siegelfähigen Packstoffs in Form von bahnförmiger Rollenware bereitgestellt. Bei dieser Ausführungsform werden die Packstoffbahnen von der Rolle abgerollt und den Stationen zugeführt, in denen die Darreichungsform auf eine der Packstoffbahnen platziert, die zweite Packstoffbahn darüber geführt und gesiegelt wird. Diese Ausführungsform ermöglicht eine besonders schnelle und zuverlässige maschinelle Verpackung und Kennzeichnung der filmförmigen Darreichungsformen.

Der siegelfähige Packstoff ist für den in der filmförmigen Darreichungsform enthaltenen Wirkstoff undurchlässig. In einer bevorzugten Ausführungsform handelt es sich bei dem siegelfähigen Packstoff um ein Laminat, umfassend eine Metallfolie, beispielsweise eine Aluminiumfolien, die auf einer ihrer beiden Flächen mit einem siegelfähigen Kunststoff beschichtet ist.

Bei dem Verfahren wird erste Lage siegelfähigen Packstoffs bereitgestellt, vorzugsweise indem die erste Lage als Bahn des siegelfähigen Packstoffs von der Rollenware abgerollt wird.

Die filmförmige Darreichungsform wird auf die mit dem siegelfähigen Kunststoff beschichtete Fläche des siegelfähigen Packstoffs platziert und mit der zweiten Lage siegelfähigen Packstoffs derart überdeckt, dass dessen mit einem siegelfähigen Kunststoff beschichtete Fläche zur folienförmigen Darreichungsform weist. Vorzugsweise wird auch die zweite Lage siegelfähigen Packstoffs als Bahn von einer Rolle abgerollt und zugeführt.

Die mit ihren mit einem siegelfähigen Kunststoff versehenen Flächen einander zugewandt und der dazwischen angeordneten filmförmigen Darreichungsform werden abschnittweise heißgesiegelt, so dass ein Siegelrandbeutel entsteht, der die filmförmige Darreichungsform enthält. "Abschnittweise" bedeutet, dass die beiden siegelfähigen Packstoffe in den Abschnitten miteinander versiegelt werden, in denen keine filmförmige Darreichungsform angeordnet ist, also entlang der Längskanten der bahnförmigen Packstoffe sowie in den Bereichen zwischen aufeinander folgende filmförmige Darreichungsformen.

Das Heißsiegeln der beiden siegelfähigen Packstoffbahnen erfolgt unter Druck und mit einer für das Heißsiegeln üblichen Temperatur von zwischen etwa 150 °C und 200 °C.

In einem weiteren Schritt des Verfahrens werden die zwischen den beiden Lagen Packstoff eingesiegelten filmförmigen Darreichungsformen mittels Blindprägen mit einem Kennzeichen versehen. Das Prägen der verpackten Darreichungsform muss mit einem Druck erfolgen, der hoch genug ist, die Darreichungsform durch die Lage Packstoff zu prägen. Der Druck darf jedoch nicht so hoch sein, dass der Packstoff beim Prägen zerstört wird.

Gemäß einer zusätzlichen und/oder alternativen Ausführungsform erfolgt das Prägen der zwischen den beiden Lagen Packstoff eingesiegelten filmförmigen Darreichungsform mit einem Druck von mindestens 2 bar, vorzugsweise mindestens 3 bar, besonders bevorzugt mindestens 4 bar, und ganz besonders bevorzugt mindestens 5 bar.

Gemäß einer zusätzlichen und/oder alternativen Ausführungsform erfolgt das Prägen der verpackten Darreichungsform mit einem Druck von nicht mehr als 10 bar, vorzugsweise von nicht mehr als 9 bar, besonders bevorzugt von nicht mehr als 8 bar, und ganz besonders bevorzugt von nicht mehr als 7 bar.

Das Prägen der filmförmigen Darreichungsform erfolgt unter Vermeidung von Wärme. Das bedeutet auch, dass die für das Prägen verwendeten Prägewerkzeuge (Matrize und/oder Patrize) nicht erwärmt werden. Gemäß einer Ausführungsform werden die für das Prägen verwendeten Prägewerkzeuge gekühlt, um einer durch die beim Prägen entstehende Erwärmung der Prägewerkzeuge entgegenzuwirken. Die Prägewerkzeuge werden vorzugsweise auf Raumtemperatur gekühlt, also auf eine Temperatur von zwischen etwa 18 °C bis etwa 23 °C. Bei einer alternativen Ausführungsform werden die Prägewerkzeuge auf eine Temperatur von etwa 4 °C gekühlt.

Das Kennzeichnen der zwischen den beiden Lagen Packstoff eingesiegelten filmförmigen Darreichungsform ist ein Blindprägen, also ein Prägen des Kennzeichens ohne Farbe.

Gemäß einer Ausführungsform ist das Blindprägen ein Planprägen. Beim Planprägen erfolgt eine Verformung des Materials (Kompression) im Bereich der Motivstelle, unter Einwirken von Druck durch den Einsatz eines Prägewerkzeugs (Prägestempel, Prägewalze, Prägeräder) als Hochdruckform. Durch die gezielt eingesetzte Druckwirkung des Prägewerkzeuges kommt es auf der geprägten Seite zu einer profilartigen Umformung des Materials, wobei die Rückseite der zwischen zwei Lagen siegelfähigen Packstoffs eingesiegelten filmförmigen Darreichungsform eben bleibt. Die rückseitige der beiden Lagen siegelfähigen Packstoffs liegt bei dem Prägevorgang auf einer ebenen Unterlage beziehungsweise Gegenwalze auf.

Gemäß einer zusätzlichen und/oder alternativen Ausführungsform weist die ebene Unterlage beziehungsweise die Gegenwalze eine elastische Fläche mit hoher Rückstellkraft auf, auf der die rückseitige der beiden Lagen siegelfähigen Packstoffs aufliegt. Bei dieser Ausführungsform kommt es auch auf der Rückseite der zwischen zwei Lagen siegelfähigen Packstoffs eingesiegelten filmförmigen Darreichungsform zu einer profilartigen Umformung des Materials. Diese Ausführungsform führt zu einer Kennzeichnung der filmförmigen Darreichungsform, die besser fühlbar ist.

Gemäß einer anderen und/oder alternativen Ausführungsform erfolgt das Prägen als Reliefprägen Bei der Reliefprägung kommt es zu einer plastischen Verformung des geprägten Materials. Dabei wird das zu prägende Material zwischen einer Prägeform (Matrize) und einer Gegenform (Patrize) unter Anwendung von Druck verdichtet. Es kommt nicht nur zu einer Verdichtung des Materials, sondern auch zu einer Dehnung des Materialgefüges an den Kanten des Motivs. Das Reliefprägen kann mittels eines Prägestempels oder einer Prägewalze erfolgen. Das Reliefprägen führt zu einer besonders ausgeprägten haptische Wirkung.

Bei der geprägten Kennzeichnung kann es sich um Zahlen, Buchstaben, graphische Symbole, Formen und Kombinationen davon handeln.

Gemäß einer Ausführungsform umfasst das Verfahren ferner das Vereinzeln individueller Siegelrandbeutel, die eine filmförmige Darreichungsform enthalten, aus einer Bahn von zwei abschnittweise miteinander versiegelten Lagen siegelfähigen Packstoffs. Das Vereinzeln der individuellen Siegelrandbeutel erfolgt durch Schneiden des Streifens oder durch Ausstanzen aus dem Streifen.

Das Vereinzeln der Siegelrandbeutel, die eine filmförmige Darreichungsform enthalten, aus der Bahn kann vor oder nach dem Prägen der eingesiegelten filmförmigen Darreichungsform erfolgen.

In einer Ausführungsform umfasst das Verfahren die folgenden Schritte:
a) Platzieren einer filmförmigen Darreichungsform zwischen zwei Lagen siegelfähigen Packstoffs;
b) abschnittweises Heißsiegeln der zwei Lagen des siegelfähigen Packstoffs zu einem die filmförmige Darreichungsform enthaltenden Siegelrandbeutel;
c) Blindprägen der in dem Siegelrandbeutel enthaltenen filmförmigen Darreichungsform durch zumindest eine der beiden Packstofflagen des Siegelrandbeutels.
d) Vereinzeln von individuellen Siegelrandbeuteln, die eine filmförmige Darreichungsform enthalten.

In einer alternativen Ausführungsform erfolgt das Prägen der verpackten Darreichungsformen vor dem Vereinzeln der individuellen Packungen. Bei dieser Ausführungsform umfasst das Verfahren die folgenden Schritte:
a) Platzieren einer filmförmigen Darreichungsform zwischen zwei Lagen siegelfähigen Packstoffs;
b) abschnittweises Heißsiegeln der zwei Lagen des siegelfähigen Packstoffs zu einem die filmförmige Darreichungsform enthaltenden Siegelrandbeutel;
c) Vereinzeln von individuellen Siegelrandbeuteln, die eine filmförmige Darreichungsform enthalten; und
d) Blindprägen der in dem Siegelrandbeutel enthaltenen filmförmigen Darreichungsform durch zumindest eine der beiden Packstofflagen des Siegelrandbeutels.

Gemäß dem zweiten Aspekt betrifft die Erfindung filmförmige Darreichungsformen, die ein Kennzeichen in Form einer Prägung aufweisen. Die filmförmige Darreichungsform liegt oder lag in einem Siegelrandbeutel vor. Die filmförmige Darreichungsform ist gemäß dem voranstehend beschriebenen Verfahren kennzeichenbar, vorzugsweise ist die filmförmige Darreichungsform mit dem vorstehend beschriebenen Verfahren

Gemäß einem Ausführungsbeispiel handelt es sich bei der filmförmigen Darreichungsform um einen stark öligen Film. Im Sinne der vorliegenden Erfindung wird unter einem stark öligen Film eine filmförmige Darreichungsform verstanden, der einen hohen Anteil an Weichmachern und/oder ätherischen Ölen enthält. Der Begriff "hoher Anteil" bezeichnet hierbei einen Anteil an öligen Substanzen, der so hoch ist, dass die Tendenz des Ausschwitzens dieser Substanz(en) aus dem Film bei Raumtemperatur unvermeidbar ist. Der absolute Gehalt an öligen Substanzen ab dem ein Ausschwitzen erfolgt, ist unter anderem abhängig vom Polymer und von der öligen Substanz. Entscheidend ist, wie gut die ölige Substanz im Polymer löslich ist.

### Ausführungsbeispiel

Ein sich im Mundraum schnell auflösender Film auf Basis eines Copolymers aus Methacrylsäure und Ethylacetat (1:1) (Eudragit® L 100-55 der Firma Evonik Nutrition Care GmbH), enthaltend 2,5 mg Nikotin und 6,64 mg Minzöl, wurde einer Blindprägung unterworfen, bei der ein Prägestempel eine zweistellige Zahl von etwa 8 mm Höhe mit einem Druck von 2 bar in den Film drückte. Nach Einsiegeln in zwei Lagen von mit siegelfähigem Kunststoff kaschierten Aluminiumfolien wurden die gekennzeichneten Filme bei Raumtemperatur gelagert, ohne gedrückt zu werden. Nach spätestens vierwöchiger Lagerzeit waren bei ausgepackten Filmen keine Prägung mehr erkennbar.

Parallel zu dem vorgenannten Kontrollexperiment wurden gleiche Filme zunächst in das gleiche Packmaterial eingesiegelt und anschließend einer Blindprägung unterworfen, bei der ein Prägestempel eine zweistellige Zahl von etwa 8 mm Höhe mit einem Druck von 2 bar durch eine Packstofflage in den Film drückte. Auch nach einer Lagerung von über einem halben Jahr war keine Veränderung in der Kennzeichnung der Filme gegenüber einer identischen Kennzeichnung unmittelbar nach dem Prägen festzustellen.

## Patentansprüche

1. Ein Verfahren zum Kennzeichnen filmförmiger Darreichungsformen, umfassend zumindest die folgenden Schritte:
- Platzieren einer filmförmigen Darreichungsform zwischen zwei Lagen siegelfähigen Packstoffs;
- abschnittweises Heißsiegeln der zwei Lagen des siegelfähigen Packstoffs zu einem die filmförmige Darreichungsform enthaltenden Siegelrandbeutel;
- Blindprägen der in dem Siegelrandbeutel enthaltenen filmförmigen Darreichungsform durch zumindest eine der beiden Packstofflagen des Siegelrandbeutels.

2. Das Verfahren gemäß Anspruch 1, wobei die zwei Lagen siegelfähigen Packstoffs in Form von bahnförmiger Rollenware bereitgestellt werden.

3. Das Verfahren gemäß Anspruch 1 oder 2, wobei das Blindprägen mit einem Druck von mindestens 2 bar und nicht mehr als 10 bar erfolgt.

4. Das Verfahren gemäß einem der Ansprüche 1 bis 3, wobei die für das Blindprägen verwendeten Prägewerkzeuge gekühlt werden.

5. Das Verfahren gemäß einem der Ansprüche 1 bis 4, wobei das Blindprägen aus der Gruppe ausgewählt ist, die Planprägen und Reliefprägen umfasst.

6. Das Verfahren gemäß einem Ansprüche 1 bis 5, wobei das Blindprägen mit einem Prägewerkzeug erfolgt, das aus der Gruppe ausgewählt ist, die eines Prägestempel, Prägeräder und Prägewalzen umfasst.

7. Das Verfahren gemäß einem der Ansprüche 1 bis 6, wobei das Kennzeichen aus der Gruppe ausgewählt ist, die Zahlen, Buchstaben, Symbole, Formen und Kombinationen davon umfasst.

8. Das Verfahren gemäß einem der Ansprüche 1 bis 7, wobei das Verfahren ferner das Vereinzeln individueller Siegelrandbeutel umfasst.

9. Das Verfahren gemäß Anspruch 8, wobei das Vereinzeln vor oder nach dem Blindprägen erfolgt.

10. Eine filmförmige Darreichungsform, die ein Kennzeichen in Form einer Prägung aufweist, gekennzeichnet mit einem Verfahren gemäß einem der Ansprüche 1 bis 9.

11. Die filmförmige Darreichungsform gemäß Anspruch 10, wobei sie in einem Siegelrandbeutel vorliegt.

12. Die filmförmige Darreichungsform gemäß einem der Ansprüche 10 oder 11, wobei die filmförmige Darreichungsform ein stark öliger Film ist.
